# EUROPÄISCHE PATENTSCHRIFT

(11) **EP 1 765 268 B1**
(45) Veröffentlichungstag und Bekanntmachung des Hinweises auf die Patenterteilung: **14.07.2010**
(21) Anmeldenummer: 05746362.2
(22) Anmeldetag: 01.06.2005
(51) Int. Cl.: A61K 8/31, A61Q 5/00, C09B 5/62

(54) **VERWENDUNG VON FLUORESZIERENDEN PERLYLENVERBINDUNGEN ZUR BEHANDLUNG MENSCHLICHER HAARE**
USE OF FLUORESCENT PERLYLENE COMPOUNDS FOR THE TREATMENT OF HUMAN HAIR
UTILISATION DE COMPOSES DE PERLYLENE FLUORESCENTS POUR TRAITER DES CHEVEUX HUMAINS

(30) Priorität: 17.06.2004 DE 102004029385
(43) Veröffentlichungstag der Anmeldung: 28.03.2007
(73) Patentinhaber: The Procter and Gamble Company, Cincinnati, OH 45202 (US)
(72) Erfinder: SPECKBACHER, Markus, 63739 Aschaffenburg (CH); BAUMEISTER, Jan, CH-1726 Farvagny-le-Grand (CH)
(74) Vertreter: Marollé, Patrick Pierre Pascal
(86) Internationale Anmeldenummer: PCT/EP2005/005860
(87) Internationale Veröffentlichungsnummer: WO 2005/123012

(56) Entgegenhaltungen:
- EP-A- 1 027 879
- EP-A- 1 502 580
- WO-A-01/92420
- WO-A-2004/033563
- DE-A1- 3 016 765
- DE-A1- 4 327 273

## Beschreibung

Gegenstand der Erfindung ist die Verwendung von fluoreszierenden Perylenverbindungen zur Behandlung menschlicher Haare, insbesondere zur Verbesserung des Haarglanzes sowie entsprechende Haarbehandlungsmittel.

Im schnelllebigen Haarstylingmarkt ist der Bedarf an Produkten mit neuen Eigenschaften sehr groß. Hohes Aufmerksamkeitspotential haben Produktinnovationen, die mehrere Sinne gleichzeitig ansprechen, positive Assoziationen wecken oder sich durch außergewöhnliche zusätzliche Eigenschaften von herkömmlichen Produkten unterscheiden. Interessant sind zum Beispiel Haarstylingprodukte, welche neben der primär gewünschten Stylingwirkung zusätzlich ungewöhnliche farbliche Wirkungen, z.B. Fluoreszenzeffekte auf menschlichem Haar zeigen oder den Haarglanz oder die Leuchtkraft der Haarfarbe verbessern. Viele bekannte Fluoreszenzfarbstoffe sind allerdings insbesondere in hydrophoben Stylingprodukten wie z.B. Haarwachsen unbefriedigend, da z.B. die Wirkung nicht ausreichend ist, oder die kosmetische Verträglichkeit oder die Verträglichkeit mit Produktinhaltstoffen nicht vollständig zufriedenstellend ist.

Von großer Bedeutung sind in der Haarkosmetik außerdem der Haarglanz menschlicher Haare und die Leuchtkraft der Haarfarben. Glänzendes Haar wird in der Regel mit gesundem, natürlichem, vitalem Haar assoziiert. Es gibt eine Reihe von Produkten zur Glanzverbesserung von Haaren. Viele Produkte, z.B. auf Basis von Ölen oder Silikonverbindungen verbessern zwar den Haarglanz, haben aber gleichzeitig nachteilige Nebenwirkungen. Insbesondere können sie das Haar belasten, was eine Anwendbarkeit auf feinem Haar erschwert. Es besteht daher ein Bedarf an weiteren, den Haarglanz, Farbeffekte auf dem Haar oder die Leuchtkraft von Haarfarben verbessernden Produkten.

EP-1027879 offenbart Haarfärbemittel, die kationische Perylenverbindungen enthalten.

Es wurde nun gefunden, dass fluoreszierende Perylenverbindungen sehr gut zur Behandlung menschlicher Haare geeignet sind, insbesondere zur Erzeugung von Glanz- und fluoreszierenden Farbwirkungen. Gegenstand der Erfindung ist daher die Verwendung von fluoreszierenden Perylenverbindungen zur kosmetischen Behandlung menschlicher Haare, insbesondere die Verwendung zur Verbesserung des Glanzes oder der Leuchtkraft menschlicher Haare.

Fluoreszierende Perylenverbindungen sind Stoffe, welche mindestens eine Perylenstruktureinheit aufweisen und in der Lage sind, Licht einer Wellenlänge, die vorzugsweise im UV-Bereich (< 400 nm) liegt, zu absorbieren und Licht einer größeren Wellenlänge, die vorzugsweise im sichtbaren Bereich liegt (> 400 nm), abzustrahlen.

Die Perylenverbindungen sind mit einer die Positionen 3,4 und/oder die Positionen 9,10 überbrückenden Gruppe substituiert, wobei die überbrückende Gruppe ausgewählt ist aus einer Gruppe -CO-NR1-CO-, wobei R1 für lineare oder verzweigte Alkylgruppen mit 1 bis 24 C-Atomen steht oder R1 ist ausgewählt aus 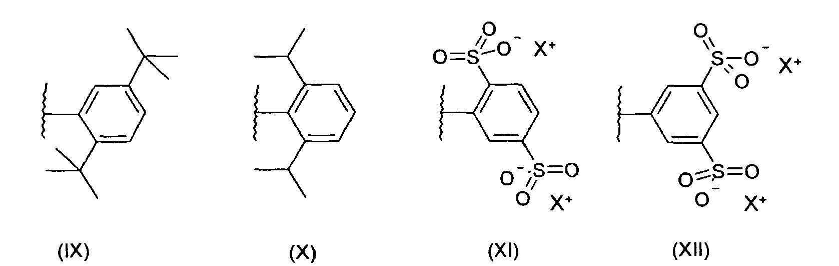 wobei X⁺ ein Proton oder ein Kation darstellt, wobei Lithiumionen, Natriumionen, Kaliumionen, Ammoniumionen, Magnesiumionen oder Calciumionen bevorzugt sind;

Die überbrückende Gruppe kann z.B. auch ausgewählt sein aus 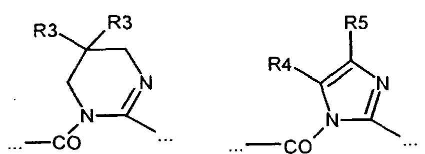 wobei R3 für eine lineare C1-C9-Alkylgruppen steht; R4 und R5 gemeinsam einen ankondensierten Kohlenwasserstoffrest bilden. Der ankondensierte Rest kann z.B. ausgewählt sein aus den Formeln

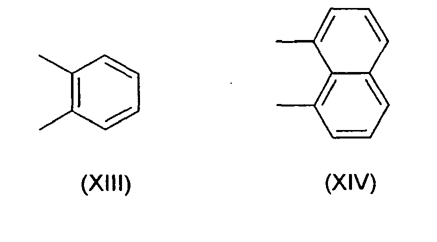

Weitere Perylenverbindungen sind solche der Formeln (I) bis (VIII) und (XIX). 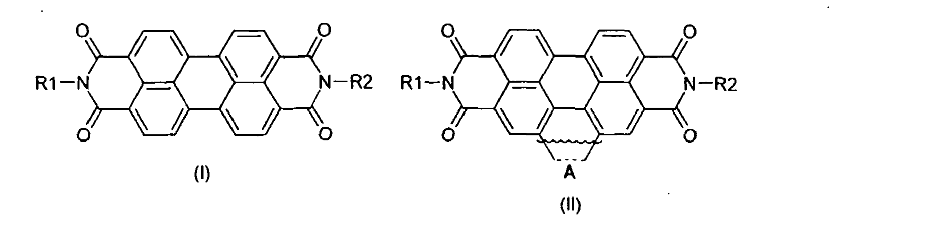 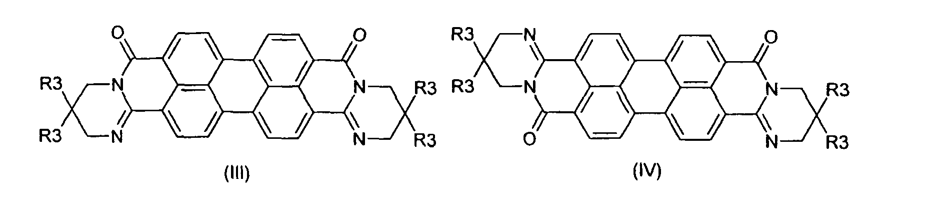 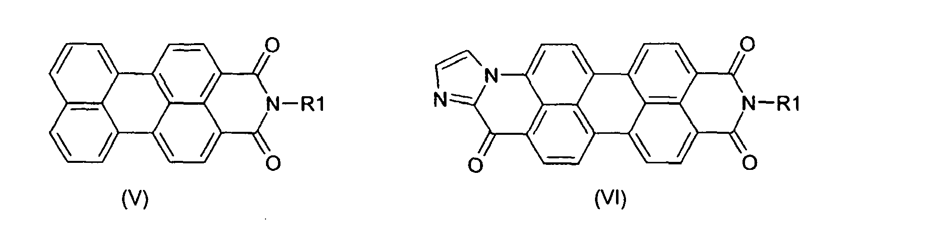 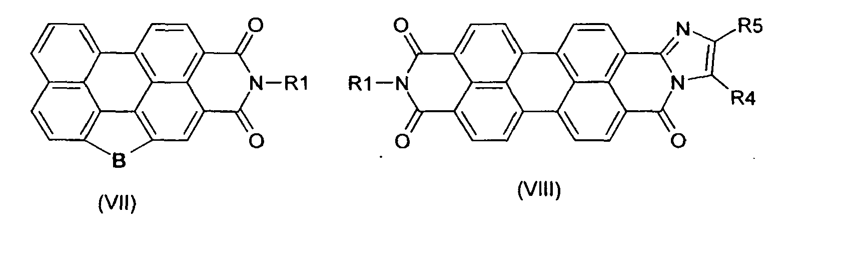 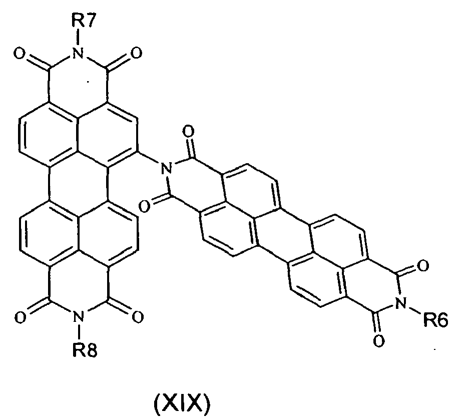 worin, R1 und R2 unabhängig voneinander für lineare oder verzweigte, unsubstituierte oder substituierte Kohlenwasserstoffgruppen, insbesondere Alkyl-, Cycloalkyl-, Aryl-, Alkylaryl- oder Arylalkylgruppen mit 1 bis 24 C-Atomen stehen oder ausgewählt werden aus Resten der Formeln (IX) bis (XII) gemäß oben genannter Definition und R3, R4 und R5 die gleiche Bedeutung haben wie oben und worin R6, R7 und R8 unabhängig voneinander für lineare oder verzweigte C₁-C₂₄-Alkylgruppen stehen.

A steht für eine divalente Verbindungsgruppe, z.B. für ein Schwefelatom, eine NH-Gruppe oder eine Verbindung ausgewählt aus der Gruppe bestehend aus den organischen Resten der Formeln (XV) bis (XVIII), 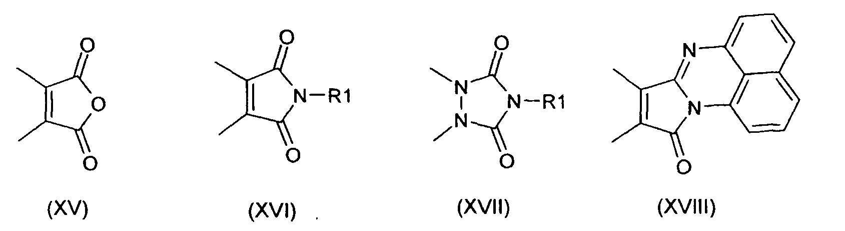 worin R₁ die vorgenannte Bedeutung hat.

B steht für eine divalente Verbindungsgruppe, z.B. für ein Schwefelatom oder eine NH-Gruppe.

Als geeignete fluoreszierende Perylenderivate der allgemeinen Formeln (I) bis (VIII) können beispielsweise genannt werden: N,N' -Bis-(alkyl)-perylen-3,4:9,10-bis(dicarboximid), z.B. N,N'-Bis-(1-butylpentyl)-perylen-3,4:9,10-bis(dicarboximid), N,N'-Bis-(1-hexylheptyl)-perylen-3,4:9,10-bis(dicarboximid), N,N`-Bis-(1-heptyloctyl)-perylen-3,4:9,10-bis(dicarboximid), N,N'-Bis-(1-octylnonyl)-perylen-3,4:9,10-bis(dicarboximid), oder N,N`-Bis-(2,5-di-tert-butylphenyl)-peryleri-3,4:9,10-bis-(dicarboximid); N-Alkyl-perylen-3,4-dicarboximid, z.B. N-(1-Hexylheptyl)-perylen-3,4-dicarboximid oder N-(2,5-Di-tert-butylphenyl)-perylen-3,4-dicarboximid; N,N'-Bis(alkyl)-benzo[ghi]perylen-2,3,8,9,11,12-hexacarbonsäure-2,3;8,9-bis(dicarboximid)-11,12-anhydrid, z.B. N,N'-Bis(1-hexylheptyl)-benzo[ghi]perylen-2,3,8,9,11,12-hexacarbonsäure-2,3;8,9-bis(dicarboximid)-11,12-anhydrid; N,N'-Bis(alkyl)-benzo[ghi]perylen-2,3,8,9,11,12-hexacarbonsäure-tris(dicarboximid), z.B. N,N`-Bis(1-hexylheptyl)-benzo-[ghi]perylen-2,3,8,9,11,12-hexacarbonsäure-tris(dicarboximid); N,N'-Bis(alkyl)-thieno[2',3',4',5':4,5]phenanthro[2,1,10-def:7,8,9-d'e'f']-diisochinolino-2H,7H-1,3,6,8-tetron, z.B. N,N'-Bis(2,5-di-tert-butylphenyl)-thieno[2',3',4',5':4,5]-phenanthro[2,1,10-def:7,8,9-d'e'f`]-diisochinolino-2*H*,7*H-*1,3,6,8-tetron und N,N'-Bis(hexylheptyl)-thieno-[2',3',4',5':4,5]phenanthro[2,1,10-def:7,8,9-d'e'f]-diisochinolino-2*H*,7*H*-1,3,6,8-tetron; wobei die Alkylgruppen jeweils für lineare oder verzweigte, unsubstituierte oder substituierte Alkyl-, Cycloalkyl-, Aryl-, Alkylaryl- oder Arylalkylgruppen mit 1 bis 24 C-Atomen stehen.

Ein geeignetes fluoreszierendes Perylenderivat der Formel (XIX) ist z.B. 1- [N^{1'}- (N^{2'}- (alkyl)-perylen-3,4:9,10-bis(dicarboximid)]-N¹,N²-bis(alkyl)-perylen-3,4:9,10-bis(dicarboximid), wobei die Alkylgruppen für lineare oder verzweigte, unsubstituierte oder substituierte Alkyl-, Cycloalkyl-, Aryl-, Alkylaryl- oder Arylalkylgruppen mit 1 bis 24 C-Atomen stehen können.

Fluoreszierende Perylenverbindungen und deren Herstellung sind an sich bekannt und werden z.B. beschrieben in DE 37 034 95; DE 196 51 712; WO 00/23446; H. Langhals, S. Kirner, "Novel Fluorescent Dyes by the Extension of the Core of Perylenetetracarboxylic Bisimides", Eur. J. Org. Chem. 2000, 365-380; H. Langhals, H. Jaschke, U. Ring, P. von Unold, "Imidazolo Perylene Imides: a Highly Fluorescent and Stable Replacement of Terrylene", Angew. Chem. 1999, 111, 143-145; Angew. Chem. Int. Ed. Engl. 1999, 38, 201-203; H. Langhals, H. Bastani-Oskoui, "Synthesis of Readily Soluble Tetraazaviolanthrone and -isoviolanthrone Fluorescent Dyes", J. Prakt. Chem. 1997, 339, 597-602; L. Feiler, H. Langhals, K. Polborn, "Synthesis of Perylene-3,4-dicarboximides - Novel, Highly Photostable Fluorescent Dyes", Liebigs Ann. Chem. 1995, 1229-1244; H. Langhals, S. Sprenger, M.-T. Brandherm, "Peryleneamidine-imide Dyes", Liebigs Ann. Chem. 1995, 481- 486; S. Demmig, H. Langhals, "Leichtlösliche, lichtechte Perylen-Fluoreszenzfarbstoffe", Chem. Ber. 1988, 121, 225-230. Auf diese Dokumente wird insoweit Bezug genommen.

Gegenstand der Erfindung sind auch die bisher nicht bekannten Perylenverbindungen der oben genannten Formel (XIX) und insbesondere 1- [N^{1'}- (N^{2'}-(alkyl)-perylen-3,4:9,10-bis(dicarboximid)]-N¹,N²-bis(alkyl)-perylen-3,4:9,10-bis(dicarboximide). Diese können beispielsweise hergestellt werden durch Kondensationsreaktion aus 1-Amino-N,N'-bis(alkyl)-perylen-3,4:9,10-bis-(dicarboximid) und N-(alkyl)-perylen-3,4:9,10-tetracarbonsäure-3,4-anhydrid-9,10-carboximid, welche an sich bekannt sind, gegebenenfalls in Gegenwart geeigneter Katalysatoren. Ein beispielhaftes Reaktionsschema mit einer 1-Hexylheptylgruppe als Alkylgruppe ist das folgende: 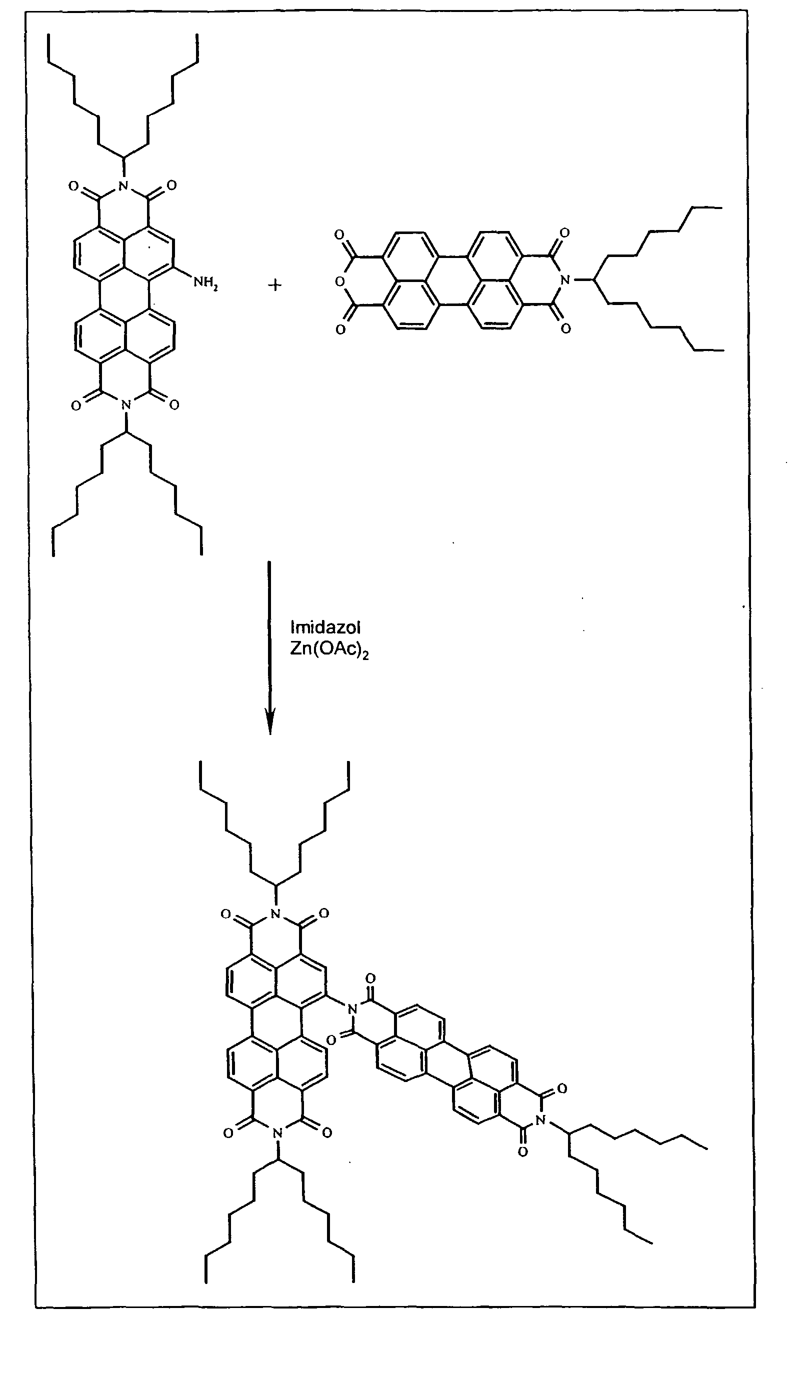 Die fluoreszierenden Perylenverbindungen können erfindungsgemäß zur Herstellung von Mitteln zur Behandlung menschlicher Haare verwendet werden. Bei den erfindungsgemäßen Haarbehandlungsmitteln kann es sich um Mittel für die Reinigung des Haares, für die Pflege des Haares oder für die temporäre Verformung und/oder Stabilisierung der Frisur (Stylingmittel) handeln, die in den unterschiedlichsten Anwendungsarten, z.B. als leave on- oder als rinse off-Produkte, appliziert werden können. Applikationsformen sind z.B. Shampoos, Haarkuren, Haarspülungen, Haarlotionen, Spitzenfluids, Haaröle, Brillantine, Haarsprays, Haarlacke, Festigerlotionen, Haarschäume, Haargele, Haarwachse, Haarcremes etc.

Erfindungsgemäße haarkosmetische Zusammensetzungen enthalten die fluoreszierenden Perylenverbindungen bevorzugt in einer Menge von 0,01 bis 20 Gew.%, insbesondere von 0,1 bis 15 Gew.% oder von 0,3 bis 10 Gew.%. Weitere Wirk- und Zusatzstoffe sind je nach Art und Einsatzzweck vorzugsweise in einer Menge von 0,01 bis 20 Gew.%, insbesondere von 0,05 bis 10 oder von 0,1 bis 5 Gew.% enthalten.

Die erfindungsgemäßen Mittel können zur Erzielung einer besonderen Leuchtkraft der damit behandelten Haare eingesetzt werden, insbesondere bei Verwendung fluoreszierender Perylenverbindungen mit hohen Quantenausbeuten (z.B. 50-100%). In einer Ausführungsform liegt das erfindungsgemäße Mittel in Form eines Haarwachses vor, d.h. es weist wachsartige Konsistenz auf und enthält mindestens einen Wachsstoff in einer Menge von vorzugsweise 0,5 bis 30 Gew.% sowie gegebenenfalls weitere wasserunlösliche Stoffe. Die wachsartige Konsistenz ist vorzugsweise dadurch gekennzeichnet, dass die Nadelpenetrationszahl (Maßeinheit 0,1 mm, Prüfgewicht 100 g, Prüfdauer 5 s, Prüftemperatur 25°C; nach DIN 51 579) größer oder gleich 10, besonders bevorzugt größer oder gleich 20 ist und dass der Erstarrungspunkt des Produktes vorzugsweise größer oder gleich 30°C und kleiner oder gleich 70°C ist, besonders bevorzugt im Bereich von 40 bis 55°C liegt.

Als Wachsstoff kann prinzipiell jedes im Stand der Technik bekannte Wachs eingesetzt werden. Hierzu zählen tierische, pflanzliche, mineralische und synthetische Wachse, mikrokristalline Wachse, makrokristalline Wachse, feste Paraffine, Petrolatum, Vaseline, Ozokerit, Montanwachs, Fischer-Tropsch-Wachse, Polyolefinwachse z.B. Polybuten, Bienenwachs, Wollwachs und dessen Derivate wie z.B. Wollwachsalkohole, Candelillawachs, Olivenwachs, Carnaubawachs, Japanwachs, Apfelwachs, gehärtete Fette, Fettsäureester, Fettsäureglyceride, wachsförmige Emulgatoren mit einem HLB-Wert unterhalb von 7, Fettalkohole, Fettsäuren oder hydrophile Wachse wie z.B. hochmolekulare Polyethylenglykole mit einem Molekulargewicht von 800 bis 20.000, vorzugsweise von 2.000 bis 10.000 g/mol, Polyethylenwachse und Silikonwachse. Die Wachsstoffe haben einen Erstarrungspunkt von vorzugsweise oberhalb 40°C, insbesondere oberhalb 55 °C. Die Nadelpenetrationszahl (0,1 mm, 100 g, 5 s, 25°C; nach DIN 51 579) liegt vorzugsweise im Bereich von 2 bis 70, insbesondere von 3 bis 40.

Zusätzlich zu den Wachsstoffen können flüssige, hydrophobe Öle enthalten sein. Die Öle haben vorzugsweise einen Schmelzpunkt von kleiner oder gleich 25°C und einen Siedepunkt von vorzugsweise über 250 °C, insbesondere über 300 °C. Hierfür kann prinzipiell jedes dem Fachmann allgemein bekannte Öl eingesetzt werden. In Frage kommen pflanzliche oder tierische Öle, Mineralöle (Paraffinum liquidum), Silikonöle oder deren Mischungen. Geeignet sind Kohlenwasserstofföle, z.B. Paraffin- oder Isoparaffinöle, Squalan, Öle aus Fettsäuren und Polyolen, insbesondere Triglyceride. Geeignete pflanzliche Öle sind z.B. Sonnenblumenöl, Kokosöl, Rizinusöl, Lanolinöl, Jojobaöl, Maisöl, Sojaöl.

In einer Ausführungsform liegt das erfindungsgemäße Mittel als Haarpflegemittel vor, z.B. als Haarkur oder als Haarlotion und enthält zusätzlich mindestens einen haarpflegenden Stoff. Der haarpflegende Stoff ist vorzugsweise in einer Menge von 0,01 bis 20, besonders bevorzugt von 0,05 bis 10, ganz besonders bevorzugt von 0,1 bis 5 Gew.% enthalten. Haarpflegende Stoffe sind z.B. ausgewählt aus kationischen Tensiden; kationischen Polymeren; Betain; Panthenol; Panthenylethylether; Sorbitol; Proteinhydrolysaten; Pflanzenextrakten oder einem der oben genannten Öle oder Wachsstoffe.

Geeignete kationische Tenside enthalten Aminogruppen oder quaternisierte hydrophile Ammoniumgruppen, welche in Lösung eine positive Ladung tragen und durch die allgemeine Formel

N⁽⁺⁾R¹R²R³R⁴ X⁽⁻⁾

dargestellt werden können, wobei R1 bis R4 unabhängig voneinander aliphatische Gruppen, aromatische Gruppen, Alkoxygruppen, Polyoxyalkylengruppen, Alkylamidogruppen, Hydroxyalkylgruppen, Arylgruppen oder Alkarylgruppen mit 1 bis 22 C-Atomen bedeuten, wobei mindestens ein Rest mindestens 6, vorzugsweise mindestens 8 C-Atome aufweist und X⁻ ein Anion darstellt, beispielsweise ein Halogen, Acetat, Phosphat, Nitrat oder Alkylsulfat, vorzugsweise ein Chlorid. Die aliphatischen Gruppen können zusätzlich zu den C-Atomen und den Wasserstoffatomen auch Querverbindungen oder andere Gruppen wie beispielsweise weitere Aminogruppen enthalten. Beispiele für geeignete kationische Tenside sind die Chloride oder Bromide von Alkyldimethylbenzylammoniumsalzen, Alkyltrimethylammoniumsalze, z.B. Cetyltrimethylammoniumchlorid oder - bromid, Tetradecyltrimethylammoniumchlorid oder -bromid, Alkyldimethylhydroxyethylammoniumchloride oder -bromide, die Dialkyldimethylammoniumchloride oder -bromide, Alkylpyridiniumsalze, beispielsweise Lauryl- oder Cetylpyridiniumchlorid, Alkylamidoethyltrimethylammoniumethersulfate sowie Verbindungen mit kationischem Charakter wie Aminoxide, z.B. Alkylmethylaminoxide oder Alkylaminoethyldimethylaminoxide. Besonders bevorzugt sind C8-22-Alkyldimethylbenzylammoniumverbindungen, C8-22- Alkyltrimethylammoniumverbindungen, insbesondere Cetyltrimethylammoniumchlorid, C8-22-Alkyldimethylhydroxyethylammoniumverbindungen, Di-(C8-22-alkyl)-dimethylammoniumverbindungen, C8-22-Alkylpyridiniumsalze, C8-22-Alkylamidoethyltrimethylammoniumethersulfate, C8-22-Alkylmethylaminoxide, C8-22-Alkylaminoethyldimethylaminoxide.

Neben den oben genannten kationischen Tensiden sind weitere geeignete kationische oder aminsubstituierte Tenside solche der Formel R1-NH-(CH₂)n-NR2R3
oder der Formel

R1-NH- (CH₂) n-N⁺R2R3R4 X⁻

worin R1 ein Acyl- oder ein Alkylrest mit 8 bis 24 C-Atomen ist, welcher verzweigt oder unverzweigt, gesättigt oder ungesättigt sein kann, wobei der Acyl- und/oder der Alkylrest eine oder mehrere OH-Gruppen enthalten kann, R2, R3 und R4 unabhängig voneinander Wasserstoff, Alkyl- oder Alkoxyalkylreste mit 1 bis 6 C-Atomen sind, welche gleich oder verschieden, gesättigt oder ungesättigt und mit einer oder mehreren Hydroxygruppen substituiert sein können, X- ein Anion ist, insbesondere ein Halogenidion oder eine Verbindung der allgemeinen Formel RSO₃⁻ , worin R die Bedeutung von gesättigten oder ungesättigten Alkylresten mit 1 bis 4 C-Atomen hat, und n eine ganze Zahl zwischen 1 und 10, vorzugsweise von 2 bis 5 bedeutet.
Vorzugsweise ist der haarpflegende Wirkstoff ein Amidoamin und/oder ein quaternisiertes Amidoamin der oben genannten Formeln, worin R1 ein verzweigter oder unverzweigter, gesättigter oder ungesättigter Acylrest mit 8 bis 24 C-Atomen ist, welcher mindestens eine OH-Gruppe enthalten kann. Bevorzugt sind auch solche Amine und/oder quaternisierte Amine, in denen mindestens einer der Reste R2, R3 und R4 ein Rest gemäß der allgemeinen Formel CH₂CH₂OR5 bedeuten, worin R5 die Bedeutung von Alkylresten mit 1 bis 4 C-Atomen, Hydroxyethyl oder H haben kann. Geeignete Amine oder Amidoamine, welche gegebenenfalls quaternisiert sein können, sind insbesondere solche mit den INCI-Bezeichnungen Ricinoleamidopropyl Betaine, Ricinoleamidopropyl Dimethylamine, Ricinoleamidopropyl Dimethyl Lactate, Ricinoleamidopropyl Ethyldimonium Ethosulfate, Ricinoleamidopropyltrimonium Chloride, Ricinoleamidopropyltrimonium methosulfate, Cocamidopropyl Betaine, Cocamidopropyl Dimethylamine, Cocamidopropyl Ethyldimonium Ethosulfate, Cocamidopropyltrimonium Chloride, Behenamidopropyl Dimethylamine, Isostearylamidopropyl Dimethylamine, Stearylamidopropyl Dimethylamine, Quaternium-33, Undecyleneamidopropyltrimonium Methosulfate.

In einer Ausführungsform enthält das erfindungsmäße Mittel als haarpflegenden oder haarfestigenden Zusatzstoff mindestens ein kationisches Polymer, d.h. ein Polymer mit kationischen oder kationisierbaren Gruppen, insbesondere primären, sekundären, tertiären oder quaternären Amingruppen in einer Menge von vorzugsweise 0,01 bis 20 Gew.% oder von 0,05 bis 10 Gew.%, besonders bevorzugt von 0,1 bis 5 Gew.%. Die kationische Ladungsdichte beträgt vorzugsweise 1 bis 7 meq/g.

Bei den geeigneten kationaktiven Polymeren handelt es sich vorzugsweise um haarfestigende oder um haarkonditionierende Polymere. Geeignete Polymere enthalten vorzugsweise quaternäre Amingruppen- Die kationischen Polymere können Homo- oder Copolymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie z.B. Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alkylvinylpyrrolidon Salze. Die Alkylgruppen dieser Monomere sind vorzugsweise niedere Alkylgruppen wie z.B. C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit quaternären Amingruppen sind z.B. die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quaternisiertes Vinylpyrrolidon/Dimethylaminoethylmethacrylat Copolymer (Polyquaternium-11) sowie quaternäre Silikonpolymere bzw. -oligomere wie beispielsweise Silikonpolymere mit quaternären Endgruppen (Quaternium-80).

Bevorzugte kationische Polymere auf synthetischer Basis: Poly(dimethyldiallylammoniumchlorid); Copolymere aus Acrylamid und Dimethyldiallylammoniumchlorid; quaternäre Ammoniumpolymere, gebildet durch die Reaktion von Diethylsulfat und einem Copolymer aus Vinylpyrrolidon und Dimethylaminoethylmethacrylat, insbesondere Vinylpyrrolidon/Dimethylaminoethylmethacrylatmethosulfat Copolymer (z.B. Gafquat^{®} 755 N, Gafquat^{®} 734); quaternäre Ammoniumpolymere aus Methylvinylimidazoliumchlorid und Vinylpyrrolidon (z.B. LUVIQUAT^{®} HM 550); Polyquaternium-35; Polyquaternium-57; Polymer aus Trimethylammonium-ethyl-methacrylatchlorid; Terpolymere aus Dimethyldiallylammoniumchlorid, Natriumacrylat und Acrylamid (z.B. Merquat^{®} Plus 3300); Copolymere aus Vinylpyrrolidon, Dimethylaminopropylmethacrylamid und Methacryloylaminopropyllauryldimethylammoniumchlorid; Terpolymere aus Vinylpyrrolidon, Dimethylaminoethylmethacrylat und Vinylcaprolactam (z.B. Gaffix^{®} VC 713); Vinylpyrrolidon/Methacrylamidopropyltrimethylammoniumchlorid Copolymere (z.B. Gafquat^{®} HS 100); Copolymere aus Vinylpyrrolidon und Dimethylaminoethylmethacrylat; Copolymere aus Vinylpyrrolidon, Vinylcaprolactam und Dimethylaminopropylacrylamid; Poly- oder Oligoester, aufgebaut aus mindestens einer ersten Monomerart, die ausgewählt ist aus mit mindestens einer quaternären Ammoniumgruppe substituierten Hydroxysäure; endständig mit quaternären Ammoniumgruppen substituierte Dimethylpolysiloxane.

Geeignete kationische Polymere, die von natürlichen Polymeren abgeleitet sind, sind insbesondere kationische Derivate von Polysacchariden, beispielsweise kationische Derivate von Cellulose, Stärke oder Guar. Geeignet sind weiterhin Chitosan und Chitosanderivate. Kationische Polysaccharide haben z.B. die allgemeine Formel

G-O-B-N⁺R^{a}R^{b}R^{c} X⁻

G ist ein Anhydroglucoserest, beispielsweise Stärke- oder Celluloseanhydroglucose;
B ist eine divalente Verbindungsgruppe, beispielsweise Alkylen, Oxyalkylen, Polyoxyalkylen oder Hydroxyalkylen;
R^{a}, R^{b} und R^{c} sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in R^{a}, R^{b} und R^{c} vorzugsweise maximal 20 ist;
X ist ein übliches Gegenanion, beispielsweise ein Halogen, Acetat, Phosphat, Nitrat oder Alkylsulfat, vorzugsweise ein Chlorid. Kationische Cellulosen sind z.B. solche mit den INCI-Bezeichnungen Polyquaternium-10 oder Polyquaternium-24. Ein geeignetes kationisches Guarderivat hat z.B. die INCI-Bezeichnung Guar Hydroxypropyltrimonium Chloride.

Besonders bevorzugte kationaktive Stoffe sind Chitosan, Chitosansalze und Chitosanderivate. Bei den erfindungsgemäß einzusetzenden Chitosanen handelt es sich um vollständig oder partiell deacetylierte Chitine. Das Molekulargewicht kann über ein breites Spektrum verteilt sein, beispielsweise von 20.000 bis ca. 5 Millionen g/mol, z.B. von 30.000 bis 70.000 g/mol. Vorzugsweise liegt das Molekulargewicht jedoch über 100.000 g/mol, besonders bevorzugt von 200.000 bis 700.000 g/mol. Der Deacetylierungsgrad beträgt vorzugsweise 10 bis 99%, besonders bevorzugt 60 bis 99%. Ein bevorzugtes Chitosansalz ist Chitosoniumpyrrolidoncarboxylat, z.B. Kytamer^{®} PC mit einem Molekulargewicht von ca. 200.000 bis 300.000 g/mol und Deacetylierung von 70 bis 85%. Als Chitosanderivate kommen quaternierte, alkylierte oder hydroxyalkylierte Derivate, z.B. Hydroxyethyl-, Hydroxypropyl- oder Hydroxybutylchitosan in Betracht. Die Chitosane oder Chitosanderivate liegen vorzugsweise in neutralisierter oder partiell neutralisierter Form vor. Der Neutralisationsgrad liegt vorzugsweise bei mindestens 50%, besonders bevorzugt zwischen 70 und 100%, bezogen auf die Anzahl der freien Basengruppen. Als Neutralisationsmittel können prinzipiell alle kosmetisch verträglichen anorganischen oder organischen Säuren verwendet werden wie beispielsweise Ameisensäure, Weinsäure, Äpfelsäure, Milchsäure, Zitronensäure, Pyrrolidoncarbonsäure, Salzsäure u.a., von denen die Pyrrolidoncarbonsäure besonders bevorzugt ist.

Bevorzugte kationische Polymere auf natürlicher Basis:
kationische Cellulosederivate aus Hydroxyethylcellulose und Diallyldimethylammoniumchlorid; kationische Cellulosederivate aus Hydroxyethylcellulose und mit Trimethylammonium substituiertem Epoxid; Chitosan und dessen Salze; Hydroxyalkylchitosane und deren Salze; Alkylhydroxyalkylchitosane und deren Salze; N-Hydroxyalkylchitosanalkylether.

In einer Ausführungsform liegt das erfindungsgemäße Mittel in Form eines Gels, einer viskosen Lotion oder in Form eines Sprühgels, welches mit einer mechanischen Vorrichtung versprüht wird, vor und enthält mindestens einen Gelbildner, vorzugsweise mindestens ein gelbildendes, verdickendes Polymer. Der Gelbildner ist in einer Menge von vorzugsweise 0,05 bis 10, besonders bevorzugt von 0,1 bis 5 Gew.% enthalten. Das gelförmige oder viskose Produkt weist eine Viskosität von vorzugsweise mindestens 250 mPa s (gemessen mit einem Bohlin Rheometer CS, Messkörper C25 bei 25°C und einer Schergeschwindigkeit von 50 s⁻¹) auf. Die Viskosität des Gels beträgt vorzugsweise von 500 bis 50.000 mPa s, insbesondere von 1.000 bis 15.000 mPa s bei 25°C.
Gelbildner sind z.B. ausgewählt aus Copolymeren aus mindestens einer ersten Monomerart, die ausgewählt ist aus Acrylsäure und Methacrylsäure und mindestens einer zweiten Monomerart, die ausgewählt ist aus Estern der Acrylsäure und ethoxyliertem Fettalkohol; vernetzter Polyacrylsäure; vernetzte Copolymere aus mindestens einer ersten Monomerart, die ausgewählt ist aus Acrylsäure und Methacrylsäure und mindestens einer zweiten Monomerart, die ausgewählt ist aus Estern der Acrylsäure mit C10- bis C30-Alkoholen; Copolymeren aus mindestens einer ersten Monomerart, die ausgewählt ist aus Acrylsäure und Methacrylsäure und mindestens einer zweiten Monomerart, die ausgewählt ist aus Estern der Itaconsäure und ethoxyliertem Fettalkohol; Copolymeren aus mindestens einer ersten Monomerart, die ausgewählt ist aus Acrylsäure und Methacrylsäure, mindestens einer zweiten Monomerart, die ausgewählt ist aus Estern der Itaconsäure und ethoxyliertem C10- bis C30-Alkohol und einer dritten Monomerart, ausgewählt aus C1- bis C4-Aminoalkylacrylaten; Copolymeren aus zwei oder mehr Monomeren, ausgewählt aus Acrylsäure, Methacrylsäure, Acrylsäureestern und Methacrylsäureestern; Copolymeren aus Vinylpyrrolidon und Ammoniumacryloyldimethyltaurat; Copolymeren aus Ammoniumacryloyldimethyltaurat und Monomeren ausgewählt aus Estern von Methacrylsäure und ethoxylierten Fettalkoholen; Hydroxyethylcellulose; Hydroxypropylcellulose; Hydroxypropylguar; Glycerylpolyacrylat; Glycerylpolymethacrylat; Copolymeren aus mindestens einem C2- C3- oder C4-Alkylen und Styrol; Polyurethanen; Hydroxypropylstärkephosphat; Polyacrylamid; mit Decadien vernetztes Copolymer aus Maleinsäureanhydrid und Methylvinylether; Johannesbrotkernmehl; Guar-Gummi; Xanthan; Dehydroxanthan; Carrageenan; Karaya-Gummi; hydrolysierte Maisstärke; Copolymere aus Polyethylenoxid, Fettalkoholen und gesättigtem Methylendiphenyldiisocyanat (z.B. PEG-150/Stearylalkohol/SMDI Copolymer).

In einer Ausführungsform liegt das erfindungsgemäße Mittel in Form eines verschäumbaren Produktes (Mousse) in Kombination mit einer Vorrichtung zum Verschäumen vor. Es enthält mindestens eine übliche, hierfür bekannte schaumgebende Substanz, z.B. mindestens ein schaumbildendes Tensid oder mindestens ein schaumbildendes Polymer. Unter Vorrichtungen zum Verschäumen sind solche Vorrichtungen zu verstehen, welche das Verschäumen einer Flüssigkeit mit oder ohne Verwendung eines Treibmittels ermöglichen. Als geeignete mechanische Schäumvorrichtung kann beispielsweise ein handelsüblicher Pumpschäumer oder ein Aerosolschaumkopf verwendet werden. Das Produkt liegt entweder in Kombination mit einer mechanischen Pumpschäumvorrichtung (Pumpschaum) oder in Kombination mit mindestens einem Treibmittel (Aerosolschaum) in einer Menge von vorzugsweise 1 bis 20, insbesondere von 2 bis 10 Gew.%, vor. Treibmittel sind z.B. ausgewählt aus Propan, Butan, Dimethylether und fluorierten Kohlenwasserstoffen. Das Mittel wird unmittelbar vor der Anwendung verschäumt und als Schaum in das Haar eingearbeitet und kann anschließend ausgespült werden oder ohne Ausspülen im Haar belassen werden.

In einer Ausführungsform liegt das erfindungsgemäße Mittel in Form eines Sprühproduktes (Haarspray) in Kombination mit einer Sprühvorrichtung vor. Es kann sich um ein Pumpspray handeln, wobei die Sprühvorrichtung eine mechanische Pumpsprühvorrichtung ist. Es kann sich auch um ein Aerosolspray handeln, wobei das Mittel in Kombination mit einer druckfesten Verpackung, einem Sprühkopf und mindestens einem Treibmittel, ausgewählt aus Propan, Butan, Dimethylether und fluorierten Kohlenwasserstoffen, vorliegt. Ein Aerosolspray enthält zusätzlich vorzugsweise 15 bis 85 Gew.%, besonders bevorzugt 25 bis 75 Gew.% eines Treibmittels und wird in einem Druckbehälter abgefüllt. Als Treibmittel sind beispielsweise niedere Alkane, wie z.B. n-Butan, i-Butan und Propan, oder auch deren Gemische sowie Dimethylether oder Fluorkohlenwasserstoffe wie F 152a (1,1-Difluorethan) oder F 134 (Tetrafluorethan) sowie ferner bei den in Betracht kommenden Drücken gasförmig vorliegende Treibmittel, wie beispielsweise N₂, N₂O und CO₂ sowie Gemische der vorstehend genannten Treibmittel geeignet.
Ein Non-Aerosol-Haarsprays wird mit Hilfe einer geeigneten mechanisch betriebenen Sprühvorrichtung versprüht. Unter mechanischen Sprühvorrichtungen sind solche Vorrichtungen zu verstehen, welche das Versprühen einer Zusammensetzung ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Das erfindungsgemäße Mittel kann auch als Shampoo in Kombination mit mindestens einem reinigungsaktiven Tensid, vorzugsweise mindestens einem anionischen, amphoteren oder nichtionischen Tensid vorliegen. Das reinigungsaktive Tensid ist in einer Menge von vorzugsweise 1 bis 50 Gew.%, vorzugsweise von 3 bis 30 Gew.%, insbesondere von 5 bis 20 Gew.% enthalten.

Geeignete anionische Tenside sind z.B. Salze und Ester von Carbonsäuren, Alkylethersulfate und Alkylsulfate, Fettalkoholethersulfate, Sulfonsäure und ihre Salze (z.B. Sulfosuccinate oder Fettsäureisethienate), Phosphorsäureester und ihre Salze, Acylaminosäuren und ihre Salze. Eine ausführliche Beschreibung dieser anionischen Tenside ist der Publikation "FIEDLER - Lexikon der Hilfsstoffe", Band 1, fünfte Auflage (2002), Seiten 97 bis 102, zu entnehmen, auf die hiermit ausdrücklich Bezug genommen wird. Geeignete anionische Tenside sind z.B. ausgewählt aus den Alkali- oder Erdalkalisalzen der C10- bis C18-Alkylsulfate, der C10- bis C18-Alkylsulfonate, der C10- bis C18-Alkylbenzolsulfonate, der C10- bis C18-Xylolsulfonate und der mit 1 bis 10 Ethylenoxideinheiten ethoxylierten C10- bis C18-Alkylethersulfate, den ethoxylierten Sulfobernsteinsäurehalbestern der Formel

R¹(OCH₂CH₂) ₘ-O₂C-CH₂CH (SO₃M)-CO₂M,

wobei R1 einen C10- bis C18-Alkylrest bedeutet, M ein Alkali- oder Erdalkalikation darstellt und m eine ganze Zahl von 1 bis 10 bedeutet; den Alkylethercarboxylaten der Formel R²(OCH₂CH₂)ₙ-OCH₂COOM, wobei R2 einen C10 bis C18-Alkylrest bedeutet, M ein Alkali- oder Erdalkalikation darstellt und n eine ganze Zahl von 1 bis 20 bedeutet. Bevorzugte Tenside sind Alkylethersulfate, Alkylsulfate und Alkylsulfonate, wobei die Alkali- und Erdalkalisalze der mit 1 bis 10 Ethylenoxideinheiten ethoxylierten C10- bis C18-Alkylethersulfate, insbesondere Natriumlaurylethersulfat besonders bevorzugt sind. Von den Alkylsulfaten ist Natriumlaurylsulfat bevorzugt. Von den geeigneten Alkylsulfonaten sind die Natriumsalze der sekundären C12- bis C16-Alkansulfonate und deren Mischungen bevorzugt. Von den geeigneten Alkylbenzolsulfonaten ist das Natriumsalz des linearen Dodecylbenzolsulfonates bevorzugt. Von den geeigneten Alkylethersulfaten ist jenes bevorzugt, das einen C12- bis C16-Alkyl-, vorzugsweise einen Laurylrest aufweist und mit 2 bis 4, vorzugsweise 3 Ethylenoxideinheiten ethoxyliert ist (INCI: Sodium Laurethsulfate). Von den geeigneten ethoxylierten Sulfobernsteinsäurehalbestern ist derjenige bevorzugt, der mit 2 bis 4, vorzugsweise 3 Ethylenoxideinheiten ethoxyliert ist und einen C12-bis C16-Alkyl-, vorzugsweise einen Laurylrest aufweist. Von den Alkylethercarboxylaten ist jenes bevorzugt, das mit 8 bis 14, vorzugsweise 10 Ethylenoxideinheiten ethoxyliert ist und einen C12- bis C16-Alkyl-, vorzugsweise einen Laurylrest aufweist.

Geeignete nichtionische Tenside sind z.B.
- Ethoxylierte Fettalkohole, Fettsäuren, Fettsäureglyceride oder Alkylphenole, insbesondere Anlagerungsprodukte von 2 bis 30 mol Ethylenoxid und/oder 1 bis 5 mol Propylenoxid an C8- bis C22-oder C12- bis C18-Fettalkohole, an C12- bis C22-Fettsäuren oder an Alkylphenole mit 8 bis 15 C-Atomen in der Alkylgruppe
- C12- bis C22-Fettsäuremono- und -diester von Anlagerungsprodukten von 1 bis 30 mol Ethylenoxid an Glycerin
- Anlagerungsprodukte von 5 bis 60 mol Ethylenoxid an Rizinusöl oder an gehärtetes (hydriertes) Rizinusöl.
- Fettsäurezuckerester, insbesondere Ester aus Saccharose und ein oder zwei C8- bis C22-Fettsäuren, INCI: Sucrose Cocoate, Sucrose Dilaurate, Sucrose Distearate, Sucrose Laurate, Sucrose Myristate, Sucrose Oleate, Sucrose Palmitate, Sucrose Ricinoleate, Sucrose Stearate
- Ester aus Sorbitan und ein, zwei oder drei C8- bis C22-Fettsäuren und einem Ethoxylierungsgrad von 4 bis 20
- Polyglycerylfettsäureester, insbesondere aus ein, zwei oder mehreren C8- bis C22-Fettsäuren und Polyglycerin mit vorzugsweise 2 bis 20 Glyceryleinheiten
- Fettsäurealkanolamide
- Alkylglukoside, Alkyloligoglukoside und Alkylpolyglucoside mit C8- bis C22-Alkylgruppen, z.B. Decyl Glucoside oder Lauryl Glucoside.

Geeignete amphotere Tenside sind z.B. Derivate aliphatischer quaternärer Ammonium-, Phosphonium- und Sulfoniumverbindungen der Formel 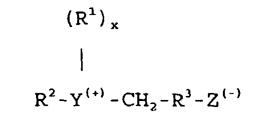 wobei R2 eine geradkettige oder verzweigtkettige Alkyl-, Alkenyl- oder Hydroxyalkylgruppe mit 8 bis 18 C-Atomen und 0 bis etwa 10 Ethylenoxideinheiten und 0 bis 1 Glycerineinheit darstellt; Y ein N-, P- oder S-Atom ist; R1 eine Alkyl- oder Monohydroxyalkylgruppe mit 1 bis 3 C-Atomen ist; X gleich 1 ist, falls Y ein Schwefelatom ist und X gleich 2 ist, wenn Y ein Stickstoffatom oder ein Phosphoratom ist; R3 eine Alkylen- oder Hydroxyalkylengruppe mit 1 bis 4 C-Atomen ist und Z⁽⁻⁾ eine Carboxylat-, Sulfat-, Phosphonat- oder Phosphatgruppe darstellt.

Andere amphotere Tenside, insbesondere Betaine sind ebenso geeignet für das erfindungsgemäße Haarreinigungsmittel. Beispiele für Betaine umfassen C8- bis C18-Alkylbetaine wie Cocodimethylcarboxymethylbetain, Lauryldimethylcarboxymethylbetain, Lauryldimethyl-alpha-carboxyethylbetain, Cetyldimethylcarboxymethylbetain, Oleyldimethylgammacarboxypropylbetain und Laurylbis-(2-hydroxypropyl)-alpha-carboxyethylbetain; C8- bis C18-Sulfobetaine wie Cocodimethylsulfopropylbetain, Stearyldimethylsulfopropylbetain, Lauryldimethylsulfoethylbetain, Lauryl-bis-(2-hydroxyethyl)sulfopropylbetain; die Carboxylderivate des Imidazols, die C8- bis C18-Alkyldimethylammoniumacetate, die C8- bis C18-Alkyldimethylcarbonylmethylammoniumsalze sowie die C8- bis C18-Fettsäurealkylamidobetaine wie z.B. das Kokosfettsäureamidopropylbetain (INCI: Cocamidopropylbetain) und das N-Kokosfettsäureamidoethyl-N-(2-(carboxymethoxy)ethyl]-glycerin (INCI: Cocoamphocarboxyglycinate). Besonders bevorzugt ist ein Tensidgemisch aus Laurylethersulfat und Cocamidopropylbetain.

In einer weiteren Ausführungsform können die fluoreszierenden Perylenderivate auch direkt als kristalliner Feststoff, z.B. als Pulver oder Granulat eingesetzt werden. Dieser Feststoff kann unmittelbar vor der Anwendung mit einer der oben gannten Applikationsformen (insbesondere Lotionen, fluiden Gelen etc.) vermischt und auf das menschliche Haar aufgebracht werden. Der vorzugsweise pulver- oder granulatförmige Feststoff kann dabei in bis zu 100% reiner Form eingesetzt werden oder er kann mit inerten Feststoffen, welche ebenfalls vorzugsweise pulver- oder granulatförmig sind, als Trägermaterial vermischt sein. Der Feststoff kann auch mit nichtflüchtigen, benetzenden Flüssigkeiten, insbesondere Ölen überzogen sein zur Vermeidung oder Unterdrückung von Staubbildung während der Anwednung. Geeignete Öle sind insbesondere hydrophobe, bei Raumtemperatur (25°C) flüssige Öle, z.B. pflanzliche oder tierische Öle, Mineralöle (Paraffinum liquidum), Silikonöle oder deren Mischungen. Geeignet sind Kohlenwasserstofföle, z.B. Paraffin- oder Isoparaffinöle, Squalan, Öle aus Fettsäuren und Polyolen, insbesondere Triglyceride. Geeignete pflanzliche Öle sind z.B. Sonnenblumenöl, Kokosöl, Rizinusöl, Lanolinöl, Jojobaöl, Maisöl, Sojaöl etc..

Die nachfolgenden Beispiele sollen den Gegenstand der Erfindung näher erläutern.

### Beispiele

In den Beispielen einzusetzende Perylenverbindungen:
1) N,N'-Bis-(1-butylpentyl)-perylen-3,4:9,10-bis(dicarboximid)
2) N,N'-Bis-(1-hexylheptyl)-perylen-3,4:9,10-bis(dicarboximid)
3) N,N'-Bis-(1-heptyloctyl)-perylen-3,4:9,10-bis(dicarboximid)
4) N,N'-Bis-(1-octylnonyl)-perylen-3,4:9,10-bis(dicarboximid)
5) N,N'-Bis-(2,5-di-tert-butylphenyl)-perylen-3,4:9,10-bis-(dicarboximid)
6) N-(1-Nexylheptyl)-perylen-3,4-dicarboximid
7) N-(2,5-Di-tert-butylphenyl)-perylen-3,4-dicarboximid
8) N,N'-Bis(1-hexylheptyl)-benzo[ghi]perylen-2;3,8,9,11,12-hexacarbonsäure-2,3;8,9-bis(dicarboximid)-11,12-anhydrid
9) N,N'-Bis(1-hexylheptyl)-benzo[ghi]perylen-2,3,8,9,11,12-hexacarbonsäure-tris(dicarboximid)
10) N,N'-Bis(2,5-di-tert-butylphenyl)-thieno-[2`,3`,4`,5`:4,5]phenanthro[2,1,10-def:7,8,9-d'e'f`]-diisochinolino-2H,7H-1,3,6,8-tetron
11) N,N'-Bis(hexylheptyl)-thieno[2',3',4',5' :4,5]-phenanthro[2,1,10-def:7,8,9-d'e'f`]-diisochinolino-2H,7H-1,3,6,8-tetron
12) 1-[N¹-(N²-(1-hexylheptyl)-perylen-3,4:9,10- bis(dicarboximid)]-N¹,N²-bis(1-hexylheptyl)-perylen-3,4:9,10-bis-(dicarboximid)

### Synthesebeispiel:

### Darstellung von 1-[N^{1'}-(N²- (1-hexylheptyl)-perylen-3, 4: 9, 10-bis(dicarboximid)]-N¹,N²-bis(1-hexylheptyl)-perylen-3,4:9,10-bis(dicarboximid)

2,17 g (2,83 mmol) 1-Amino-N,N'-bis(1-hexylheptyl)-perylen-3,4:9,10-bis-(dicarboximid)¹⁾, 1,77 g (3,10 mmol) N-(1-hexylheptyl)-perylen-3,4:9,10-tetracarbonsäure-3,4-anhydrid-9,10-carboximid²⁾, 0,128 g (0,82 mmol) Zinkacetatdihydrat und 15 g Imidazol werden 45 Minuten bei 150 °C gerührt. Nach dem Abkühlen wird der Ansatz mit 50 ml Ethanol aus dem Reaktionsgefäss gespült, in 250 ml 2 N Salzsäure gegossen und noch eine Stunde lang bei Raumtemperatur gerührt. Der ausgefallene Farbstoff wird über eine Glasfritte abgesaugt und im Vakuum bei 40 °C getrocknet. Die Reinigung erfolgt säulenchromatographisch über Kieselgel mit Toluol als Laufmittel. Das Produkt wird dabei als hellrot leuchtende Bande als konzentrierte Fraktion eluiert. Verunreinigungen, vor allem das im Überschuss eingesetzte N-(1-hexylheptyl)-perylen-3,4:9,10-tetracarbonsäure-3,4-anhydrid-9,10-carboximid, besitzen geringere R_{f}-Werte und können somit leicht vom Hauptprodukt abgetrennt werden. Die Produktfraktion, die dünnschichtchromatographisch einheitlich erscheint, wird am Rotationsverdampfer aufkonzentriert, nach dem Abkühlen langsam mit kaltem Methanol ausgefällt, abgesaugt und getrocknet.
Ausbeute: 2,40 g (64%) rotes Pulver mit Smp. > 300 °C.
UV/Vis (Chloroform): ₘₐₓ = sh 466.4 nm, 492.9, sh 525.0, 529.7.
1) H. Langhals, S. Kirner, Eur. J. Org. Chem. 2000, 365-380.
2) H. Kaiser, J. Lindner, H. Langhals, Chem. Ber. 1991, 124, 529-535.

### Beispiel 1: Haarspülung

| | |
|---|---|
| 4,3 g | Cetearylalkohol (Lanette^{®} O) |
| 0,4 g | Cetyllactat |
| 0,5 g | Vaseline |
| 1,2 g | Cetyltrimethylammoniumchlorid |
| 0,45 g | Polyvinylpyrrolidon |
| 0,3 g | Perylenverbindung 1, 2, 3, 4, 5, 6, 7, 8, 9, 10, 11 und/oder 12 |
| ad 100 g | Wasser |

### Beispiel 2: Haarkur

| | |
|---|---|
| 5,5 g | Cetearylalkohol (Lanette^{®} O) |
| 1,2 g | Vaseline |
| 1,0 g | Paraffinum Liquidum |
| 0,5 g | Dimethylpolysiloxan (Belsil^{®} DM 500) |
| 0,3 g | Lanolinalkohol |
| 0,2 g | Lanolin |
| 1,2 g | Cetyltrimethylammoniumchlorid |
| 0,3 g | Zitronensäure |
| 0,4 g | Parfüm |
| 0,3 | Perylenverbindung 1, 2, 3, 4, 5, 6, 7, 8, 9, 10, 11 und/oder 12 |
| ad 100 g | Wasser |

### Beispiel 3: Cremeförmige Haarkur

| | |
|---|---|
| 6 g | Cetearylalkohol |
| 1,7 g | Glycerin |
| 1 g | Cetyltrimethylammoniumchlorid |
| 1 g | Pflanzenöl |
| 0,5 g | Panthenol |
| 0,5 g | Silikonöl (Dimethylpolysiloxan) |
| 0,2 g | Parfüm |
| 0,3 g | Perylenverbindung 1, 2, 3, 4, 5, 6, 7, 8, 9, 10, 11 und/oder 12 |
| Ad 100 g | Wasser |

### Beispiel 4: Leave-on Sprüh-Haarkur

| | |
|---|---|
| 0,5 g | Cetylalkohol |
| 0,3 g | Glycerin |
| 0,25g | Cetyltrimethylammoniumchlorid |
| 0,2 g | Styrol/Vinylpyrrolidon Copolymer |
| 0,2 g | Panthenol |
| 0,3 g | Perylenverbindung 1, 2, 3, 4, 5, 6, 7, 8, 9, 10, 11 und/oder 12 |
| 0,2 g | Parfüm |
| 4,8 g | Ethanol |
| Ad 100 g | Wasser |

### Beispiel 5: Haarwachs

| | |
|---|---|
| 10 g | Triceteareth-4 Phosphate |
| 6 g | PEG-200 Hydrogenated Glyceryl Palmate |
| 18 g | Caprylic/Capric Triglyceride |
| 0,5 g | Parfüm |
| 0,5 g | Konservierungsmittel |
| 0,1 g | Perylenverbindung 1, 2, 3, 4, 5, 6, 7, 8, 9, 10, 11 und/oder 12 |
| ad 100 g | Wasser |

### Beispiel 6: Haarshampoo

| | |
|---|---|
| 30 g | Natriumlaurylethersulfat |
| 8 g | Cocamidopropylbetain |
| 3 g | Glykoldistearat |
| 0,5 g | Parfüm |
| 0,35 g | Natriumbenzoat |
| 0,15 g | Natriumformiat |
| 0,2 g | Natriumchlorid |
| 0,1 g | Perylenverbindung 1, 2, 3, 4, 5, 6, 7, 8, 9, 10, 11 und/oder 12 |
| ad 100 | g Wasser |

### Beispiel 7: Creme-Duschbad

| | |
|---|---|
| 12 g | 70%iges Natriumlaurylethersulfat |
| 0,7 g | Konservierungsmittel |
| 0, 4 g | Parfüm |
| 7,5 g | Trübungsmittel (20 Gew.% PEG-3 Distearat und 18 Gew.% Natriumlaurylethersulfat in Wasser) |
| 0,3 g | Polyquaternium-10 (kationische Cellulose) |
| 0,4 g | PEG-7 Glyceryl Cocoat |
| 0,3 g | Perylenverbindung 1, 2, 3, 4, 5, 6, 7, 8, 9, 10, 11 und/oder 12 |
| 0,3 g | D-Panthenol |
| 5 g | Cocamidopropylbetain, 30%ig in Wasser |
| 1 g | NaCl |
| ad 100 g | Wasser |

## Patentansprüche

1. Verwendung von fluoreszierenden Perylenverbindungen zur kosmetischen Behandlung menschlicher Haare, **dadurch gekennzeichnet, dass** die Perylenverbindung mit einer die Positionen 3,4 und/oder die Positionen 9,10 überbrückenden Gruppe substituiert ist, wobei die überbrückende Gruppe ausgewählt ist aus
-CQ-NR1-CO-; 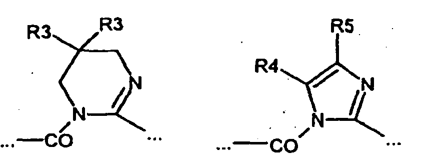 wobei R1 für lineare oder verzweigte C1-C24-Alkylgruppen steht oder ausgewählt ist aus 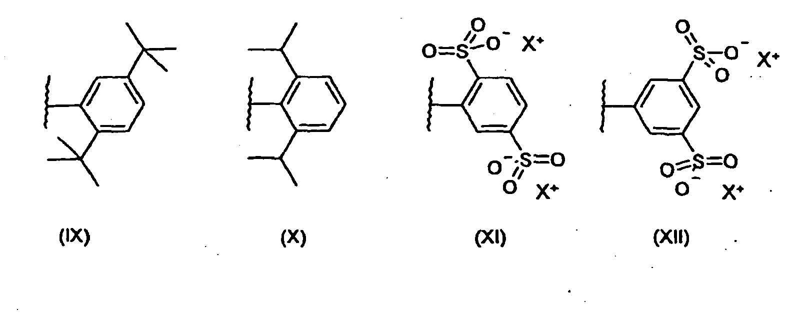 wobei X' ein Proton oder ein Kation darstellt; R3 für eine lineare C1-C9-Alkylgruppe steht; R4 und R5 gemeinsam einen ankondensierten Kohlenwasserstoffrest bilden.

2. Verwendung nach Anspruch 1 zur Verbesserung des Glanzes oder der Leuchtkraft menschlicher Haare.

3. Verwendung nach einem der vorhergehenden Ansprüche, **dadurch gekennzeichnet, dass** die Perylenverbindung ausgewählt ist aus 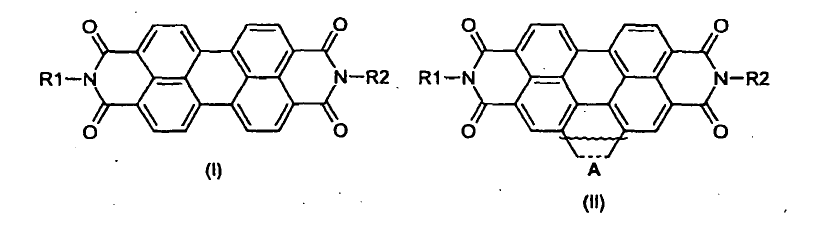 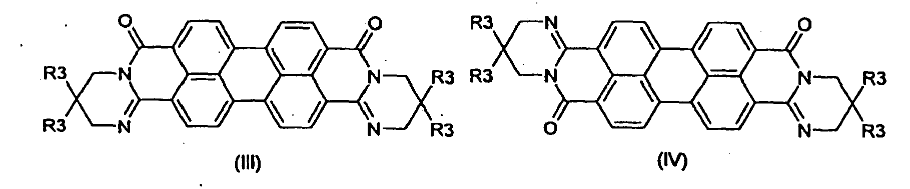 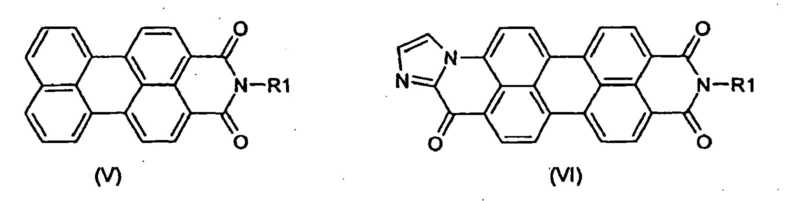 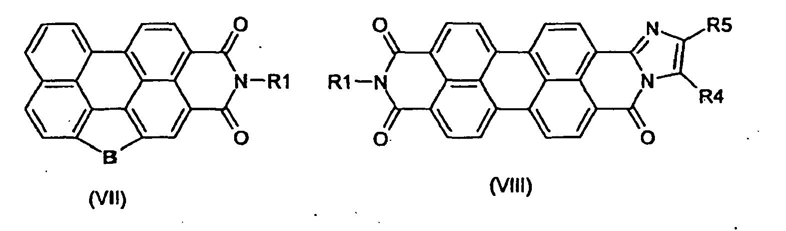 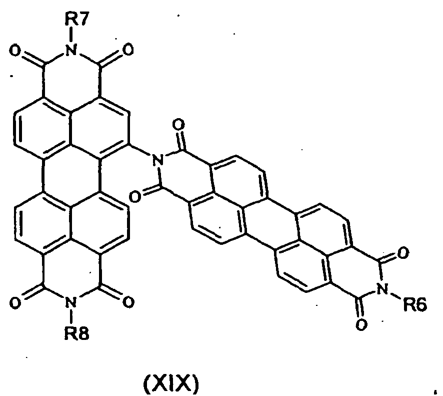 worin, R1 und R2 unabhängig voneinander für lineare oder verzweigte C₁-C₂₄-Alkylgruppen stehen oder ausgewählt werden aus Resten der allgemeinen Formeln (IX) bis (XII) gemäß Anspruch 1 und R3, R4 und R5 die gleiche Bedeutung haben wie in Anspruch 1 und worin R6, R7 und R8 unabhängig voneinander für lineare oder verzweigte C₁-C₂₄-Alkylgruppen stehen und A und B für divalente Verbindungsgruppen stehen.

4. Verwendung nach einem der vorhergehenden Ansprüche, **dadurch gekennzeichnet, dass** die Perylenverbindung ausgewählt ist aus N,N`-Bis-(alkyl)-perylen-3,4:9,10-bis(dicarboximid), N-Alkyl-perylen-3,4-dicarboximid, N,N`-Bis(alkyl)-benzo(ghi]-perylen-2,3,8,9,11,12-hexacarbonsäure-2,3;8,9-bis(dicarboximid)-11,12-anhydrid, N,N'-Bis(alkyl)-benzo[ghi]perylen-2,3,8,9,11,12-hexacarbonsäure-tris(dicarboximid), N,N'-Bis(alkyl)-thieno(2`,3`,4`,5`:4,5]phenanthro[2,1,10-def:7,B,9-d'e'f'] -diisochinolino'-2*H*,7*H*-1,3,6,8-tetron, 1-[N^{1'}-(N^{2'}-(alkyl)-perylen-3,4:9.10-bis(dicarboximid)]-N¹,N²-bis(alkyl)-perylen-3,4:9,10-bis(dicarboximid).

5. Verwendung einer fluoreszierenden Perylenverbindung nach einem der vorhergehenden Ansprüche zur Herstellung eines Mittels zur Behandlung menschlicher Haare, wobei das Mittel ausgewählt ist aus Haarwachsen, Haarkuren, Haarlotionen, Haargelen, Haarcremes, Haarschäumen, Haarsprays und Shampoos.

6. Haarbehandlungsmittel, welches zur Behandlung menschlicher Haare geeignet ist, mindestens eine fluoreszierende Perylenverbindung enthält und entweder
- als Haarwachs vorliegt mit einem Gehalt an mindestens einem Wachsstoff oder
- als Haarkur oder Haarlotion in Form einer mindestens einen zusätzlichen, haarpflegenden Stoff enthaltenden Zubereitung vorliegt oder
- als Haargel mit mindestens einem Gelbildner vorliegt oder
- als Aerosol- oder Pumpschaum in Kombination mit einer Vorrichtung zum Verschäumen vorliegt oder
- als Haarspray in Kombination mit einer Sprühvorrichtung vorliegt oder
- als Shampoo vorliegt mit einem Gehalt an mindestens einem reinigungsaktiven Tensid.

7. Perylenverbindung der allgemeinen Formel 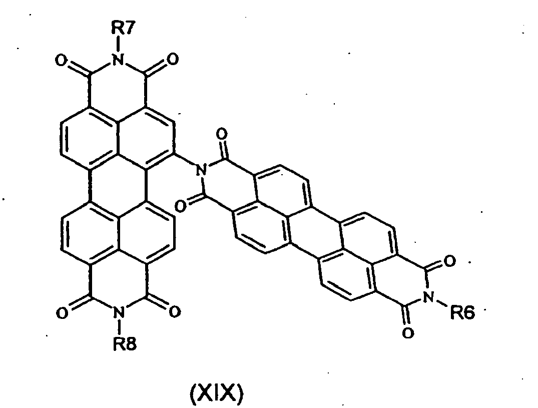 worin R6, R7 und R8 unabhängig voneinander für lineare oder verzweigte C₁-C₂₄-Alkylgruppen stehen.

8. 1-[N^{1'}-(N^{2'}-(alkyl)-perylen-3, 4:9,10-bis(dicarboximid)] - N¹,N²-bis(alkyl)-perylen-3, 4: 9, 0-bis (dicarboximid).

## Claims

1. Use of fluorescent perylene compounds for the cosmetic treatment of human hair, **characterized in that** the perylene compound is substituted by a group bridging positions 3, 4 and/or positions 9, 10, wherein the bridging group is selected from
-CO-NR1-CO-; 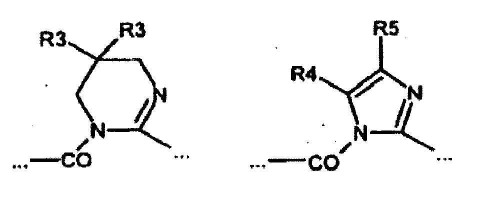 in which R1 represents linear or branched C1-C24 alkyl groups or is selected from 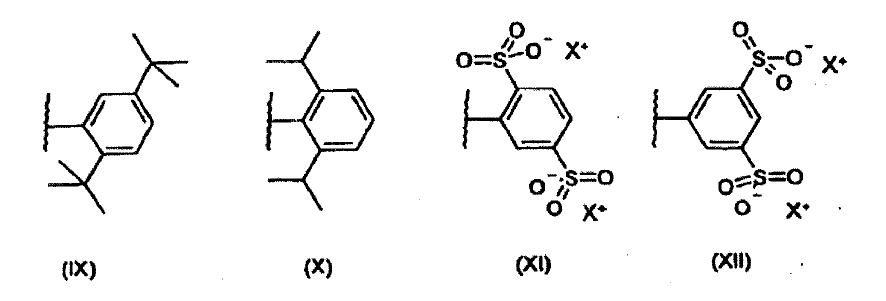 in which X⁺ is a proton or a cation; R3 is a linear C1-C9 alkyl group; R4 and R5 together form a fused-on hydrocarbon radical.

2. Use according to claim 1 for improving the shine or the luminosity of human hair.

3. Use according to one of the preceding claims, **characterized in that** the perylene compound is selected from 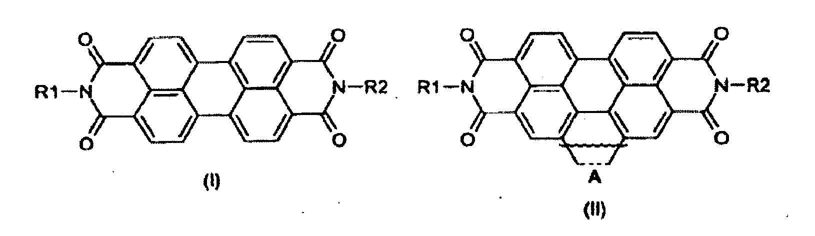 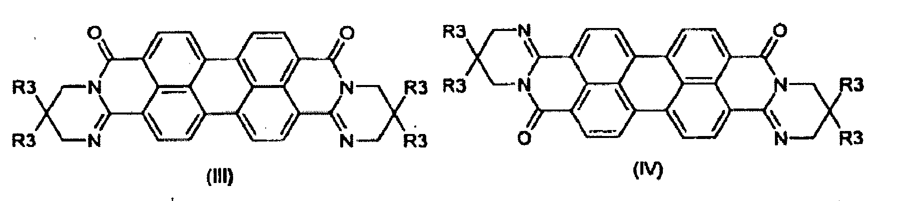 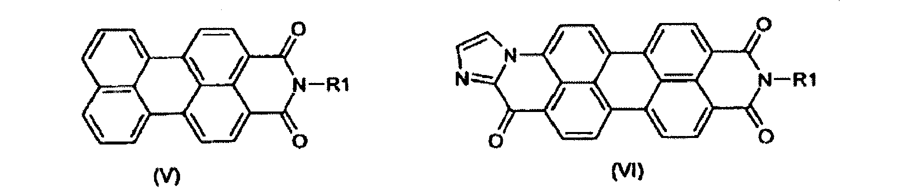 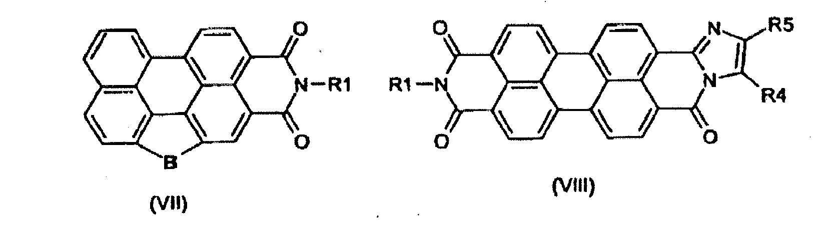 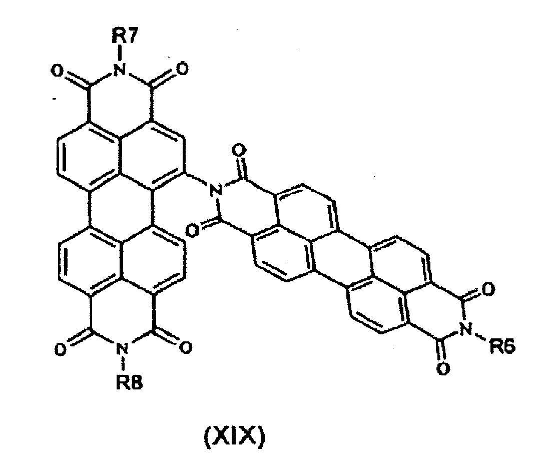 in which R1 and R2 independently of one another are linear or branched C₁-C₂₄ alkyl groups or are selected from radicals of general formulae (IX) to (XII) according to claim 1, and R3, R4 and R5 have the same meaning as in claim 1, and in which R6, R7 and R8 independently of one another are linear or branched C₁-C₂₄ alkyl groups, and A and B are divalent connecting groups.

4. Use according to one of the preceding claims, **characterized in that** the perylene compound is selected from N,N'-bis(alkyl)perylene-3,4:9,10-bis(dicarboximide), N-alkylperylene-3,4-dicarboximide, N,N'-bis(alkyl)benzo-[ghi]perylene-2,3,8,9,11,12-hexacarboxylic 2,3;8,9-bis-(dicarboximide)-11,12-anhydride, N,N'-bis(alkyl)benzo[ghi]-perylene-2,3,8,9,11,12-hexacarboxylic tris(dicarboximide), N,N'-bis(alkyl)thieno-[2',3',4',5':4,5]phenanthro[2,1,10-def:7,8,9-d'e'f']-diisoquinolino-2H,7H-1,3,6,8-tetrone, 1-[N^{1'}-(N^{2'}-(alkyl)perylene-3,4:9,10-bis(dicarboximide)]-N¹,N²-bis(alkyl)perylene-3,4:9,10-bis(dicarboximide).

5. Use of a fluorescent perylene compound according to one of the preceding claims for producing an agent for treating human hair, wherein the agent is selected from hair waxes, hair treatments, hair lotions, hair gels, hair creams, hair mousses, hair sprays and shampoos.

6. Hair treatment agent which is suitable for treating human hair, contains at least one fluorescent perylene compound and exists either
- as a hair wax containing at least one wax substance or
- as a hair treatment or hair lotion in the form of a preparation containing at least one additional hair care substance or
- as a hair gel containing at least one gel former or
- as an aerosol or pump mousse in combination with a foaming device or
- as a hair spray in combination with a spraying device or
- as a shampoo containing at least one cleaning-active surfactant.

7. Perylene compound of general formula 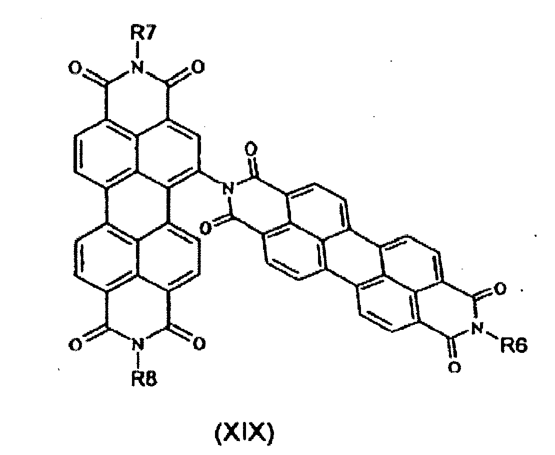 in which R6, R7 and R8 independently of one another are linear or branched C₁-C₂₄ alkyl groups.

8. 1-[N^{1'}-(N^{2'}- (Alkyl)perylene-3,4:9,10-bis-(dicarboximide)]-N¹,N²-bis(alkyl)perylene-3,4:9,10-bis(dicarboximide).

## Revendications

1. Utilisation de composés de pérylène fluorescents pour le traitement cosmétique des cheveux humains, **caractérisée en ce que** le composé de pérylène est substitué par un groupe formant un pont sur les positions 3, 4 et/ou les positions 9, 10, dans laquelle le groupe formant un pont est choisi parmi
-CO-NR1-CO- ; 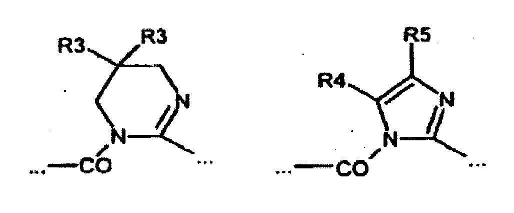 dans laquelle R1 représente des groupes alkyle linéaires ou ramifiés en C1 à C24 ou est choisi parmi 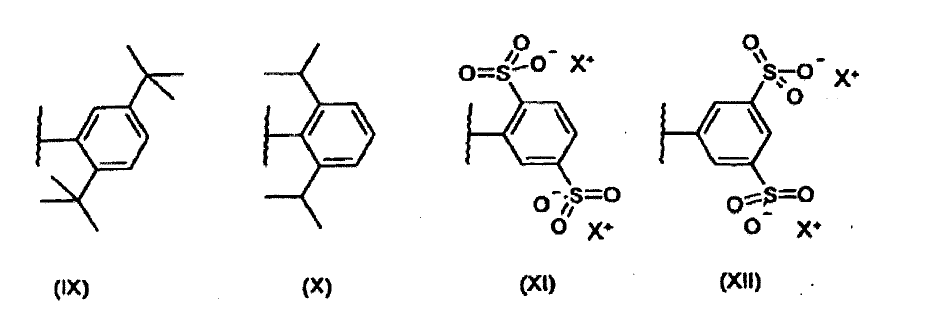 dans laquelle X⁺ est un proton ou un cation ; R3 est un groupe alkyle linéaire en C1 à C9 ; R4 et R5 forment ensemble un radical hydrocarbure fusionné.

2. Utilisation selon la revendication 1 pour améliorer la brillance ou la luminosité des cheveux humains.

3. Utilisation selon l'une des revendications précédentes, **caractérisée en ce que** le composé de pérylène est choisi parmi 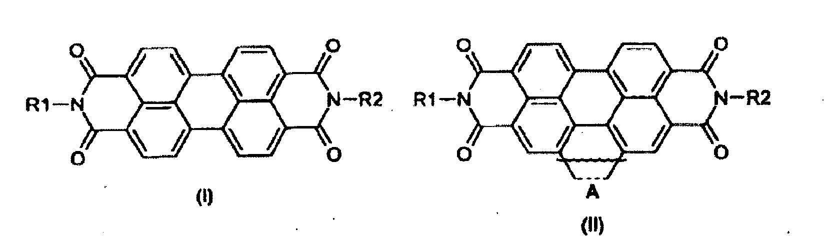 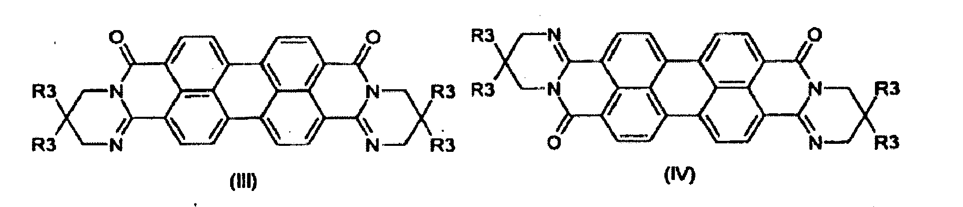 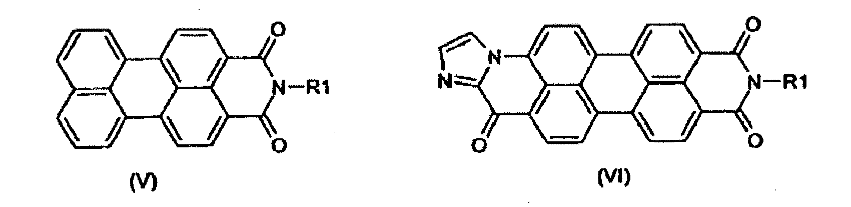 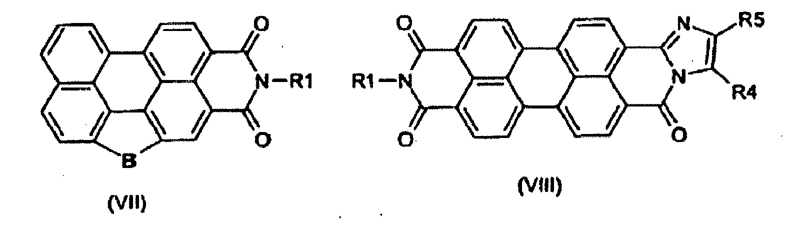 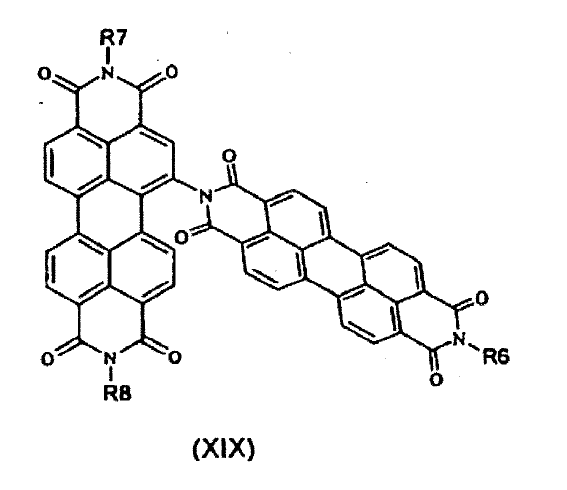 où R1 et R2 indépendamment l'un de l'autre sont des groupes alkyle linéaires ou ramifiés en C₁ à C₂₄ ou sont choisis parmi des radicaux de formule générale (IX) à (XII) selon la revendication 1, et R3, R4 et R5 ont la même signification que dans la revendication 1, et où R6, R7 et R8 indépendamment l'un de l'autre sont des groupes alkyle linéaires ou ramifiés en C₁ à C₂₄, et A et B sont des groupes de raccordement divalents.

4. Utilisation selon l'une des revendications précédentes, **caractérisée en ce que** le composé de pérylène est choisi parmi N,N'-bis(alkyl)pérylène-3,4:9,10-bis(dicarboximide), N-alkylpérylène-3,4-dicarboximide, N,N'-bis(alkyl)benzo[ghi]pérylène-2,3,8,9,11,12-hexacarboxylique 2,3;8,9-bis(dicarboximide)-11,12-anhydride, N,N'-bis(alkyl)benzo[ghi]pérylène-2,3,8,9,11,12-hexacarboxylique tris(dicarboximide), N,N'-bis(alkyl)thiéno-[2',3',4',5':4,5]phénanthro[2,1,10-def-7,8,9-d'e'f]-diisoquinolino-2H,7H-1,3,6,8-tétrone, 1-[N^{1'}-(N^{2'}-(alkyl)pérylène-3,4:9,10-bis(dicarboximide)]-N¹,N²-bis(alkyl)pérylène-3,4:9,10-bis(dicarboximide).

5. Utilisation d'un composé de pérylène fluorescent selon l'une des revendications précédentes pour fabriquer un agent pour le traitement des cheveux humains, dans laquelle l'agent est choisi parmi les cires capillaires, les traitements capillaires, les lotions capillaires, les gels pour cheveux, les crèmes pour cheveux, les mousses pour cheveux, les aérosols pour cheveux et les shampooings.

6. Agent de traitement capillaire qui est approprié pour le traitement des cheveux humains, contient au moins un composé de pérylène fluorescent et existe ou
- en tant que cire capillaire contenant au moins une substance de type cire ou
- en tant que traitement capillaire ou lotion capillaire sous la forme d'une préparation contenant au moins une substance de soin capillaire supplémentaire ou
- en tant que gel pour cheveux contenant au moins un formateur de gel ou
- en tant que mousse en aérosol ou à pompe en combinaison avec un dispositif de moussage ou
- en tant qu'aérosol pour les cheveux en combinaison avec un dispositif de pulvérisation ou
- en tant que shampooing contenant au moins un agent tensioactif actif pour le nettoyage.

7. Composé de pérylène de formule générale 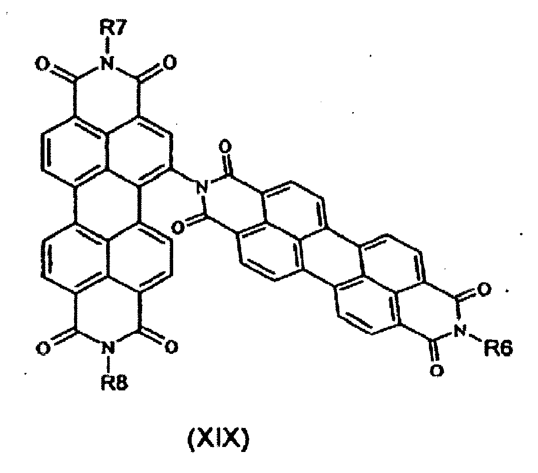 dans laquelle R6, R7 et R8 indépendamment l'un de l'autre sont des groupes alkyle linéaires ou ramifiés en C₁ à C₂₄.

8. 1-[N^{1'}-(N^{2'}-(alkyl)pérylène-3,4:9,10-bis(dicarboximide)]-N¹,N²-bis(alkyl)pérylène-3,4:9,10-bis(dicarboximide).
